# EUROPEAN PATENT APPLICATION

(11) **EP 3 808 440 A1**
(43) Date of publication of application: **21.04.2021**
(21) Application number: 19203431.2
(22) Date of filing: 15.10.2019
(51) Int. Cl.: B01F 13/08, B01F 13/00, B01F 3/04, B01F 15/00, C12M 1/02

(54) **DEVICE FOR AGITATING A LIQUID AND HOLDER FOR A MAGNETIC AGITATOR**

(71) Applicant: Technische Universität München, 80333 München (DE)
(72) Inventor: Woortman, Dirk Volker, 85356 Freising (DE); Brück, Thomas, 85452 Eichenried (DE)
(74) Representative: Koplin, Moritz

(57) **Abstract**

Provided is a holder 10 for a magnetic agitator **12** which comprises a top section **14** which is configured to be attached to a vessel **18**, wherein the top section **14** is preferably configured to be attached to an aperture **16** of said vessel, a bottom section **24** which is configured to be immersed into a liquid **28** contained in said vessel **18**, and an elongate middle section **26** arranged between and connecting said bottom section **24** and said top section **14**, wherein said bottom section **24** forms at least one of a cage **30** and an annular guide **30a** for said magnetic agitator **12**.

## Description

### Technical Field

The present disclosure relates to a holder for a magnetic agitator. In particular, the present disclosure relates to a bioreactor which allows agitating a liquid and dispersing a gas into the agitated liquid.

### Background Art

Bioreactors may be used to cultivate light-responsive cells in a liquid. Constantly agitating said liquid ensures a uniform distribution of cells in said liquid and prevents an inhomogeneous distribution of nutrients and CO₂. This may be necessary to ensure that all cells are cultivated under the same favorable conditions that foster cell growth.

### Summary

The present disclosure provides:
- a holder for a magnetic agitator;
- a device for agitating a liquid contained in a vessel and dispersing a gas into said agitated liquid, wherein the device comprises the holder; and
- methods of cultivating light-responsive cells in a liquid, which involve using the holder for agitating the liquid.

The holder comprises a top section which is configured to be attached to a vessel (e.g., the top section may be configured to be attached to an aperture of said vessel), a bottom section which is configured to be immersed into a liquid contained in said vessel, and an elongate middle section arranged between and connecting said bottom section and said top section, wherein said bottom section forms at least one of a cage and an annular guide for said magnetic agitator.

In this regard, the terms "top section", "middle section", and "bottom section", as used throughout the description and the claims, refer to positions of said sections relative to each other, when the holder is attached to the vessel and the bottom section is immersed into the liquid. When the holder is attached to the vessel, the bottom section is below the middle section and the top section is above the middle section.

Moreover, the term "vessel", as used throughout the description and the claims, particularly refers to an open-topped cylindrical container or a container with a cylindrical neck. Furthermore, the formulation "attached to an aperture of said vessel", as used throughout the description and the claims, particularly refers to an arrangement where said top section is attached to a rim of the vessel. For example, said top section may be sitting on the rim or said top section may be placed within the neck of the vessel and provided with fasteners which extend over the rim.

Furthermore, the term "elongate", as used throughout the description and the claims, particularly refers to a structure which has a height that is larger than the length or the width of the structure. Moreover, the term "cage", as used throughout the description and the claims, particularly refers to a structure with two (or more) openings, which encloses a radially symmetric chamber. For example, the cage may comprise two outer ring-shaped sections and an inner ring-shaped section, wherein the outer ring-shaped sections have a smaller inner diameter than the inner ring-shaped section, and the magnetic agitator may have a length or an outer diameter that is substantially equal to the inner diameter of the inner ring-shaped section. For instance, the magnetic agitator may be a magnetic element which is slidably supported within the cage, such as a rod, a bar or an impeller. In addition, the term "annular guide", as used throughout the description and the claims, particularly refers to a structure that guides the magnetic agitator along a closed path.

This increases the running smoothness of the magnetic agitator but does not require attaching the magnetic agitator to a drive shaft. Attaching the magnetic agitator to a drive shaft would necessitate sealing the drive shaft to avoid contamination of the liquid, thereby increasing the effort required to manufacture the holder.

The holder may further comprise a channel for feeding a gas into said agitated liquid.

For example, CO₂ or another gas may be fed into said liquid by connecting a gas pipe to the channel and controlling a flow of gas through the channel. Controlling a flow of gas through the channel may involve opening and/or closing a valve. For example, the pressure within the gas pipe may be held constant and the pressure within the channel may be controlled by opening and/or closing the valve.

Said gas may be fed into said agitated liquid through at least one of a porous material and a ring-shaped element made of a porous material.

For example, an outlet of the channel may be provided with the porous material, and the ring-shaped porous material may be sandwiched between two outer ring-shaped elements, wherein the outer ring-shaped elements form openings which allow the liquid to flow through the cage.

In this regard, the term "porous material", as used throughout the description and the claims, shall be construed broadly and particularly includes any structure forming a multitude of channels, wherein the size of the channels and the properties of the material are such that the material is substantially impermeable to the liquid but permeable to the gas.

The holder may further comprise means for generating a rotating electromagnetic field for causing a rotation of said magnetic agitator.

For example, the holder may comprise electromagnets, wherein the electromagnets and the magnetic agitator form an electric motor, or the holder may comprise a permanent magnet (in addition to the magnetic agitator) which is caused to rotate. For instance, a rotation of the permanent magnet could be controlled via a mechanic drive, e.g., a pneumatic or a hydraulic drive. A drive shaft of the mechanic drive may be integrated, at least section-wise, into the elongate middle section such that no sealing is required. Similarly, the pneumatic or hydraulic channels may be integrated, at least section-wise, into the elongate middle section. For example, the elongate middle section may comprise a hollow structure and the drive may be embedded into said hollow structure.

If the magnetic agitator is an impeller, the impeller may cause a vertical flow of the liquid. For example, the impeller may cause a flow through said cage and/or said annular guide towards the bottom of the vessel and the gas dispersed at the outer side of said cage and/or said annular guide may create an airlift updraft which results in homogeneous mixing.

An axis through a center of rotation of said rotating electromagnetic field may extend through a center of said cage and/or said annular guide.

This may further increase the running smoothness of the magnetic agitator. Moreover, the inner wall of said cage may inhibit a horizontal movement of the center of rotation of the magnetic agitator.

Said elongate middle section may comprise at least one of a hollow bar and a hollow rod.

This may decrease a weight of the middle section.

At least one of said channel and a line for supplying energy to said means for generating a rotating electromagnetic field may be arranged, at least section-wise, within said hollow bar or said hollow rod.

For example, an electric wire may be arranged and extend through said hollow bar or said hollow rod. Moreover, said hollow bar or said hollow rod may be used as a pneumatic or hydraulic channel.

Said top section may comprise a sensor socket for holding a sensor in a position in which said sensor is immersed in said liquid.

For example, the sensor socket may comprise an aperture in the top section, wherein the sensor sits on a rim of said aperture or an outer thread of the sensor engages with an inner thread of said aperture.

Said top section may comprise a disc-shaped member and said sensor socket may be formed at a center of said disc-shaped member.

For example, the sensor may be a sensor for measuring the acidity or alkalinity of the liquid and placing the sensor socket at the center of the disc-shaped member may ensure that the sensor measures the acidity or alkalinity of the liquid at a position which lies on a central axis (axis of symmetry) of the vessel.

The holder may further comprise an at least partially transparent hollow elongate member, wherein said at least partially transparent hollow elongate member is configured to be attachable to and detachable from said top section and said at least partially transparent hollow elongate member extends from said top section towards said bottom section when said at least partially transparent hollow elongate member is attached to said top section.

In this regard, the term "hollow elongate member", as used throughout the description and the claims, particularly refers to a cylindrical open-topped container such as, for example, a sample tube. Furthermore, the formulation "at least partially transparent", as used throughout the description and the claims, particularly refers to a member which is made of a transparent material such as (acrylic) glass and shall include members with one or more opaque surface areas. Moreover, the formulation "configured to be attachable to and detachable from said top section", as used throughout the description and the claims, particularly refers to a scenario where no tools are needed for attaching and detaching a member from the remainder of the holder.

Said at least partially transparent hollow elongate member may extend through said top section.

For example, said at least partially transparent hollow elongate members may be inserted into and extend through apertures in the top section, wherein a rim of an aperture serves as a holder for a member or said aperture has an inner thread which engages with an outer thread of the member.

The holder may further comprise a cap which is configured to be attachable to and detachable from said top section, wherein said cap comprises a detector which is arranged within said at least partially transparent hollow elongate member when said cap is attached to said top section.

That is, instead of immersing the detector into the liquid, the detector may be separated from the liquid that surrounds the detector. As a result, the liquid will not be contaminated by substances on the detector surface and hence, the detector may not have to be (extensively) cleaned/sterilized (e.g., in an autoclave).

The cap may comprise circuitry for monitoring and/or analyzing sensor measurements and for controlling actors such as the magnetic agitator (or a valve) based on the analysis. In other words, the cap may only require a connection to a power supply (and a gas supply) for the whole device to function properly.

The holder may further comprise another at least partially transparent hollow elongate member, wherein said other at least partially transparent hollow elongate member is configured to be attachable to and detachable from said top section, wherein said other at least partially transparent hollow elongate member extends from said top section towards said bottom section when said other at least partially transparent hollow elongate member is attached to said top section.

Said other at least partially transparent hollow elongate member may extend through said top section and said cap may comprise a source of electromagnetic radiation.

Said source of electromagnetic radiation may be arranged within said other at least partially transparent hollow elongate member when said cap is attached to said top section.

That is, instead of immersing a source of electromagnetic radiation (e.g., a light source) into the liquid, the source of electromagnetic radiation may be separated from the liquid (that surrounds the source of electromagnetic radiation) by the other at least partially transparent hollow elongate member. As a result, the liquid will not be contaminated by substances on the surface of the source of electromagnetic radiation and the source of electromagnetic radiation may not have to be (extensively) cleaned/sterilized (e.g., in an autoclave).

Said detector may be mounted to a first side of a printed circuit board which comprises another source of electromagnetic radiation mounted to a second side of said printed circuit board, and said source of electromagnetic radiation may be mounted to a second side of another printed circuit board which comprises another detector mounted to a first side of said other printed circuit board.

Said printed circuit boards may each comprise two detectors. Said detectors may be mounted to the same side or to opposite sides of a printed circuit board. The detectors mounted to one printed circuit board may be arranged and configured to measure electromagnetic radiation incident from different directions each such that one detector is directly exposed to the electromagnetic radiation emitted by a source of electromagnetic radiation, whereas the other detector (only) detects scattered electromagnetic radiation. The required directivity characteristics of the detectors may be achieved by using lenses, filters, mirrors and/or shields.. Notably, a printed circuit board may function as a shield (due to its relative orientation). The orientation of adjacent printed circuit boards (with source-detector-sets) may be fixed or variable (during or between measurements). The required directivity characteristics may also be achieved by suitable detector orientation.

The directivity characteristics allow independently measuring direct and scattered electromagnetic radiation. Moreover, the emission of electromagnetic radiation of the sources of electromagnetic radiation may be coordinated to allow distinguishing between backscattered and side scattered electromagnetic radiation. For example, electromagnetic radiation may be emitted alternatingly by the sources of electromagnetic radiation such that the detectors alternatingly detect backscattered and side scattered electromagnetic radiation. Or, the sources of electromagnetic radiation may emit electromagnetic radiation of different wavelengths (e.g., non-overlapping spectra). This may allow distinguishing between backscattered and side scattered electromagnetic radiation based on the different spectra. Being able to differentiate between transmittance, backscatter and side scatter influence enables a more accurate (non-linear) turbidity/optical density estimation.

Said holder may comprise at least three substantially identical at least partially transparent hollow elongate members and said three substantially identical at least partially transparent hollow elongate members may be arranged at corners of an equilateral polygon lying in a horizontal plane. Each of said at least partially transparent hollow elongate members may house a detector and a source of electromagnetic radiation, preferably one detector and two sources of electromagnetic radiation.

Thus, the sources of electromagnetic radiation and the detectors may form a measurement ring or a ring-like measurement setup.. Hardware-based or software-based averaging of respective transmittance, backscatter and side scatter measurements taken by different detectors of the measurement ring or the ring-like measurement setup allows avoiding or reducing a measurement bias that would otherwise occur under anisotropic conditions.

The device for agitating a liquid contained in a vessel and dispersing a gas into said agitated liquid may comprise a vessel and said holder, wherein said holder is attached to an aperture of said vessel.

A first method of cultivating light-responsive cells may comprise providing a vessel with a liquid and said light-responsive cells, immersing at least one of said cage and said annular guide of said holder into said liquid, agitating said liquid in said vessel by generating a rotating magnetic field and dispersing a gas into said agitated liquid. Said gas may comprise CO₂ and a flow rate of the gas may be controlled based on an acidity or alkalinity of the liquid.

A second method of cultivating light-responsive cells may comprise providing a vessel with a liquid and said light-responsive cells, immersing at least one of said cage and said annular guide of said holder into said liquid, placing said detector within said at least partially transparent elongate hollow member by attaching said cap to said top section, agitating said liquid in said vessel by generating a rotating magnetic field, dispersing a gas into said agitated liquid and detecting an intensity of electromagnetic radiation incident on the detector. The measured turbidity/optical density may be used for growth rate and/or cell concentration estimation during cultivation.

Moreover, the measured turbidity/optical density may be used to control the luminance. For example, light-responsive cells may be cultivated at constant light intensity. I.e., the transmittance, backscatter and side scatter measurements may be used to determine a luminance required to keep the light intensity within the fluid constant (despite the decreasing transmittance of the suspension). Moreover, some or all substances (CO₂, nutrients, etc.) required for cell growth may be automatically monitored and replenished.

The estimated growth rate and/or cell concentration may also be used to control further aspects of the cultivation. For example, ultra-violet, near-infrared or visible light may be used to evoke and control different reactions (at once or in sequence) within the cells or the cultivation.

Furthermore, a current gas flow rate may be determined based on a signal noise pattern. If the gas comprises CO₂, the flow rate of the gas may be controlled based on an acidity or alkalinity of the liquid. However, if the acidity or alkalinity of the liquid is not known, a desired gas flow rate may be derived from a heuristic and the noise pattern may be used to adapt the current gas flow rate to what is desired.

It will be appreciated that the features and attendant advantages of the disclosed holder/device may be realized by the disclosed methods and vice versa. Moreover, it is noted that throughout the description, features in brackets are to be regarded as optional.

### Brief Description of Drawings

The foregoing aspects and many of the attendant advantages will become more readily appreciated as the same becomes better understood by reference to the following description of embodiments, when taken in conjunction with the accompanying drawings, wherein like reference numerals refer to like parts throughout the various views, unless otherwise specified.
Fig. 1a illustrates a holder for a magnetic agitator.
Fig. 1b illustrates a modification of the holder of Fig. 1a.
Fig. 1c illustrates a further modification of the holder of Fig. 1a or Fig. 1b
Fig. 2a illustrates additional elements of the holder shown in Fig. 1a, Fig. 1b and Fig. 1c.
Fig. 2b illustrates a modification of the holder of Fig. 2a.
Fig. 3a shows a cross-sectional view of the bottom and middle sections of the holder of Fig. 2a and Fig. 2b.
Fig. 3b illustrates a modification of the bottom section of the holder of Fig. 3a.
Fig. 4a is a perspective view of the holder of Fig. Fig. 2b.
Fig. 4b illustrates a modification of the holder of Fig. Fig. 4a.
Fig. 4c illustrates another modification of the holder of Fig. Fig. 4a.
Fig. 5 illustrates the holder of Fig. 2b, wherein the holder comprises a sensor socket and (at least partially transparent) hollow elongate members.
Fig. 6 illustrates a holder which comprises a cap.
Fig. 7 illustrates sections of printed circuit boards which may be placed within the (at least partially transparent) hollow elongate members.
Fig. 8 shows a flow-chart of a first process of cultivating light-responsive cells.
Fig. 9 shows a flow-chart of a second process of cultivating light-responsive cells.

Notably, the drawings are not drawn to scale and unless otherwise indicated, they are merely intended to conceptually illustrate the structures and procedures described herein.

### Description of Embodiments

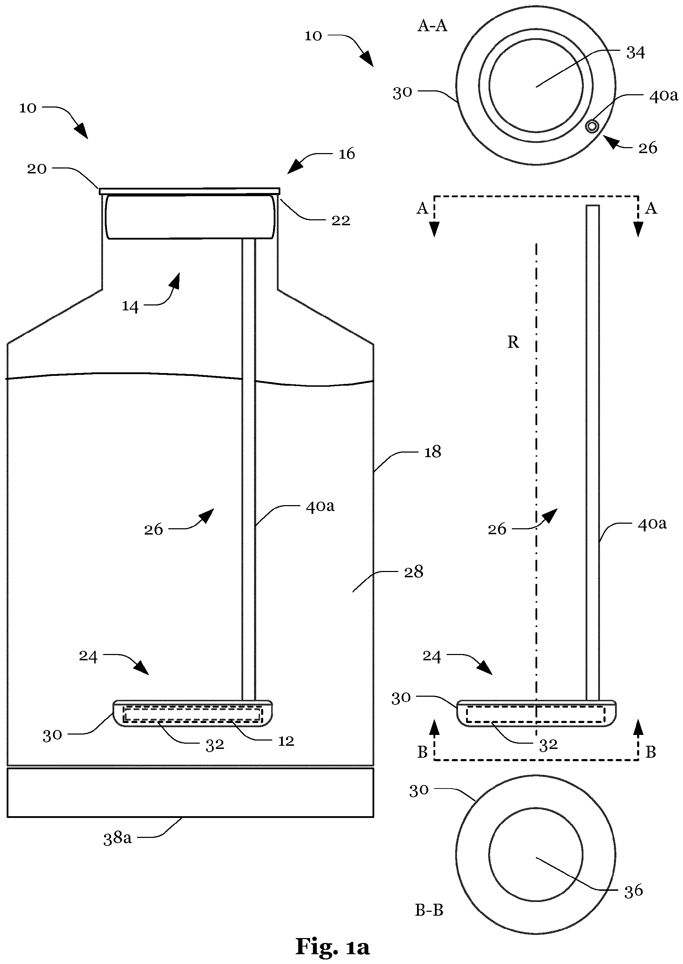

Fig. 1a shows side, top and bottom views of a holder 10 for a magnetic agitator 12. The holder 10 comprises a top section 14 which, as illustrated in the side view on the left-hand side of Fig. 1a, is attached to an aperture 16 of a vessel 18 by a disc-shaped member 20 which sits on a rim 22 of the aperture 16. The holder 10 further comprises a bottom section 24 which is connected to the top section 14 by an elongate middle section 26 that is arranged between the top section 14 and the bottom section 24.

The bottom section 24 is immersed into a liquid 28 contained in the vessel 18 and forms a cage 30 for the magnetic agitator 12. The cage 30 has a ring-like shape, wherein the symmetry axis of said ring is parallel to a longitudinal axis of the elongate middle section 26. The top section 14 has a cylinder-like shape and the symmetry axis of said cylinder is parallel to the longitudinal axis of the elongate middle section 26. The symmetry axes of the cylinder and the ring are colinear.

The vessel 18 has a volume of about 1 dm3, but larger or smaller vessels 18 are contemplated. The vessel 18 may, at least partially, be made of a transparent material such that electromagnetic radiation/light from a source of electromagnetic radiation/light arranged outside of the vessel 18 may enter the vessel 18. The cage 30 encloses a radially symmetric chamber 32 with a first opening 34 and a second opening 36. An axis R which extends vertically through a center of the first opening 34 and through a center of the second opening 36 may be colinear with a symmetry axis of the vessel 18. Notably, the elongate middle section 26 is horizontally offset from the axis R.

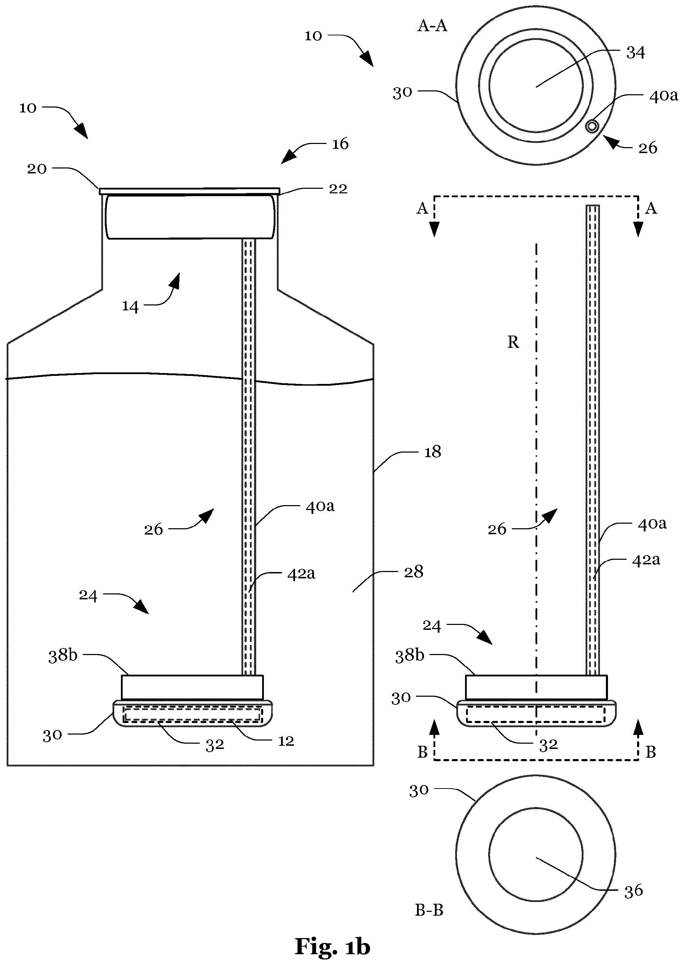

When in use, the vessel 18 may be placed on a stirrer device 38a which generates a rotating magnetic field that causes the magnetic agitator 12 to rotate within the cage 30. A center of rotation of the rotating magnetic field may lie on the axis R. As shown in Fig. 1b, a magnetic stirrer device 38b may be integrated into the holder 10 (or even into the cage 30 as will be shown further below). The magnetic stirrer device 38b may comprise electromagnets (not shown), wherein the electromagnets and the magnetic agitator 12 form an electric motor. Moreover, the holder 10 may comprise a permanent magnet (not shown) (in addition to the magnetic agitator 12) which is caused to rotate by mechanical force. For instance, the permanent magnet could be caused to rotate by a mechanic drive, e.g., a pneumatic or a hydraulic drive.

A line (not shown) for supplying electric or kinetic energy to the stirrer device 38b may be integrated into the elongate middle section 26. As illustrated in the side view in Fig. 1b on the right-hand side, the elongate middle section 26 may comprise a hollow rod 40a and supply lines (if any) may extend through the hollow rod 40a. Notably, the elongate middle section 26 may also take on other forms such as a hollow bar (not shown) or another hollow elongate member. The hollow rod 40a, hollow bar or other hollow elongate member may be welded to the cage 30. Moreover, the hollow rod 40a, hollow bar or other hollow elongate member may be used for feeding a gas into the agitated liquid 28. I.e., a channel 42a for feeding a gas into the agitated liquid 28 may be integrated into/extend through the hollow rod 40a, hollow bar or other hollow elongate member. To improve dispersion of said gas within the agitated liquid 28, a porous material (not shown) may be arranged at an outlet of the channel 42a.

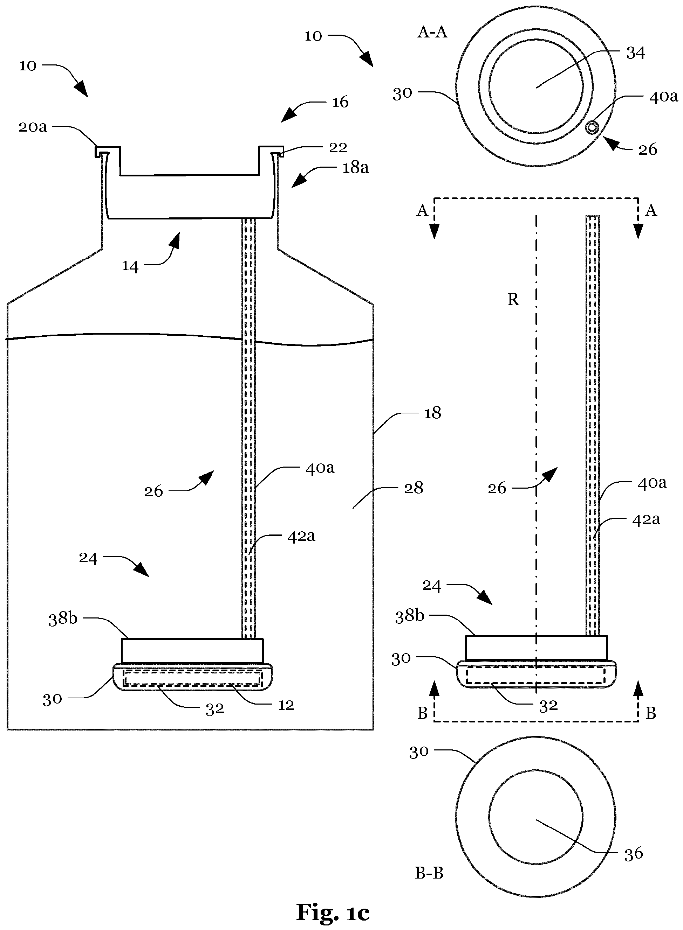

Fig. 1c shows a modification of the holder 10, wherein the disc-shaped member 20 which sits on the rim 22 of the aperture 16 is replaced by fasteners 20a which extend over the rim 22. The fasteners 20a are formed as arms which extend from the top section 14 (which is arranged within the neck 18a) radially over the rim 22. As will become more apparent from the following discussion, moving the top section 14 towards the center of the vessel 18 allows reducing the length of the elongate middle section 26 and decreases how much elements on top of the top section 14 extend vertically over the rim 22.

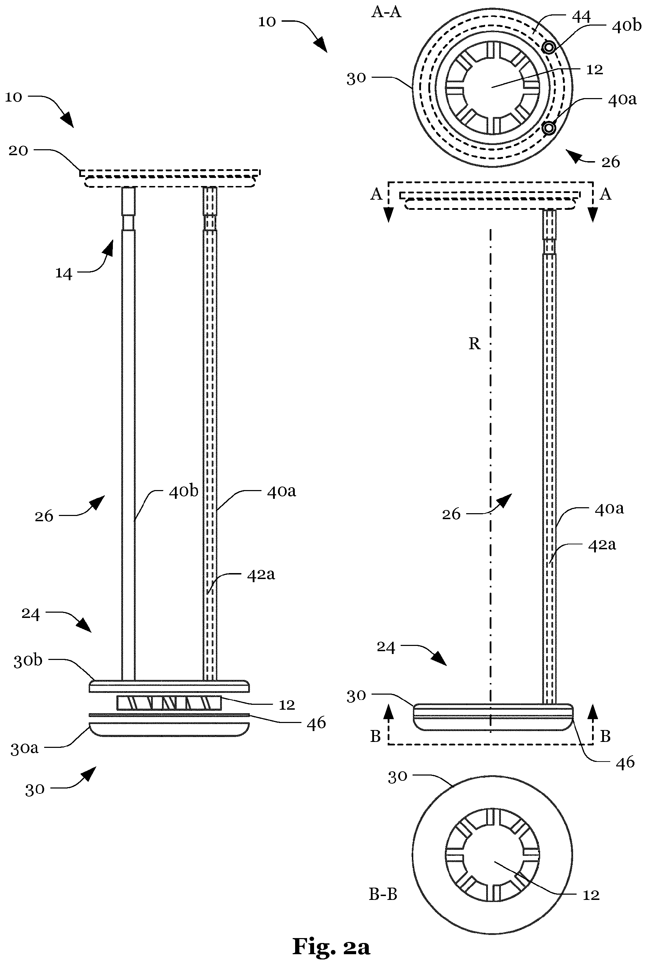

As shown in Fig. 2a, the cage 30 may comprise a ring-shaped channel section 44 made of a porous material. The porous material may comprise Polytetrafluoroethylene (PTFE) or Polyethylene (PE). A gas fed into the ring-shaped channel section 44 may pass through a ring-shaped element 46 which may be placed between two outer ring-shaped elements 30a and 30b of the cage 30. The outer ring-shaped elements 30a and 30b of the cage 30 may be fastened to each other (for example, the outer ring-shaped elements 30a and 30b of the cage 30 may be screwed to each other). The gas that passes through the porous material may cause gas bubbles at an outer surface and at an inner surface of the ring-shaped element 46, and the gas bubbles (which may have a diameter of below 100 µm) may be dispersed into the agitated liquid 28.

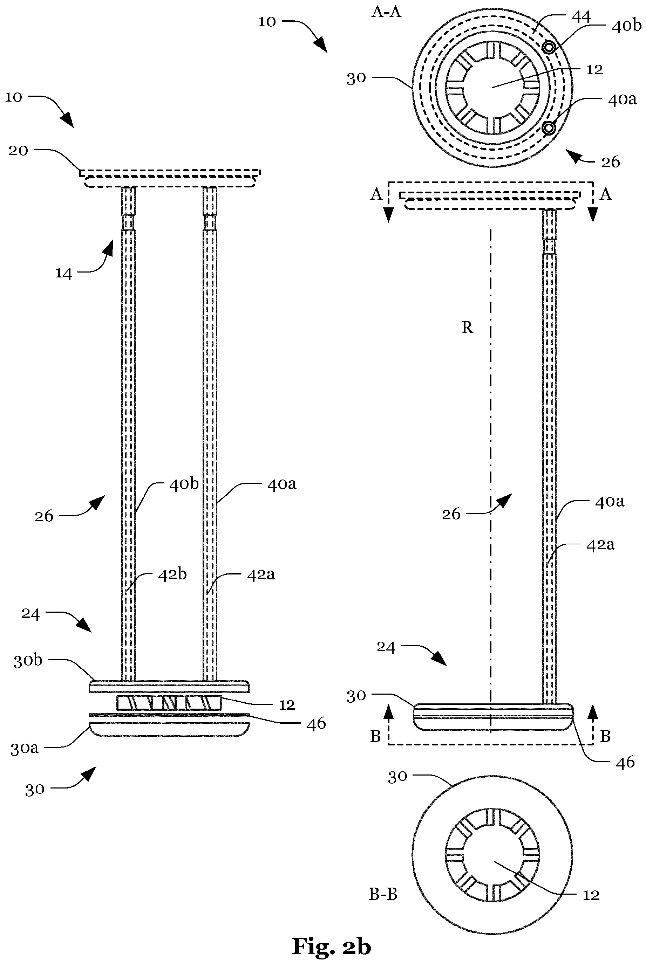

As illustrated in Fig. 2a, the magnetic agitator 12 may be an impeller causing the liquid 28 to flow through the cage 30 (along the inner surface) and around the cage 30 (along the outer surface). As discussed in connection with Fig. 1a and Fig. 1b, the impeller may be driven by an external stirrer device 38a or an internal stirrer device 38b. Furthermore, as illustrated in Fig. 2a, the elongate middle section 26 may comprise a further hollow rod 40b, a further hollow bar (not shown) or a further hollow elongate member. This may increase the rigidity of the holder 10. Furthermore, as illustrated in Fig. 2b, the further hollow rod 40b may also be used as a gas channel 42b, or for feeding (electric or kinetic) energy to an internal stirrer device 38b as shown in Fig. 1b and Fig. ic.

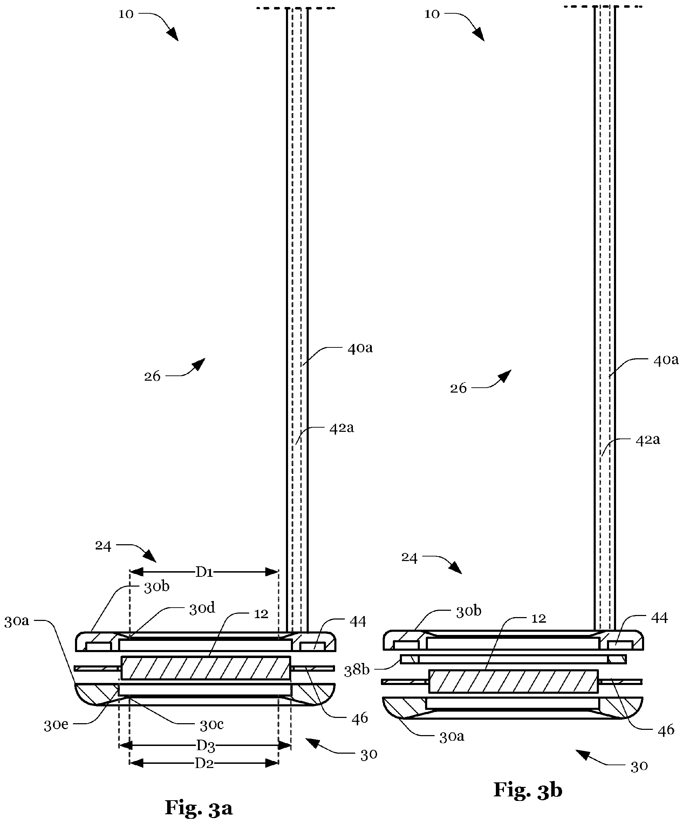

Fig. 3a shows a cross-sectional view of the bottom section 24 and the elongate middle section 26 of the holder 10 of Fig. 2a and Fig. 2b. The cage 30 comprises two outer ring-shaped sections 30c and 30d and an inner ring-shaped section 30e, wherein inner diameters D1 and D2 of the outer ring-shaped sections 30c, 30d are smaller than an inner diameter D3 of the inner ring-shaped section 30e. The magnetic agitator 12 has an outer diameter that is substantially equal to the inner diameter of the inner ring-shaped section 30e, such that the center of the magnetic agitator 12 remains in place when agitating the liquid 28. Fig. 3b shows a modification of the holder of Fig. 3a, where the stirrer device 38b is integrated into the cage 30.

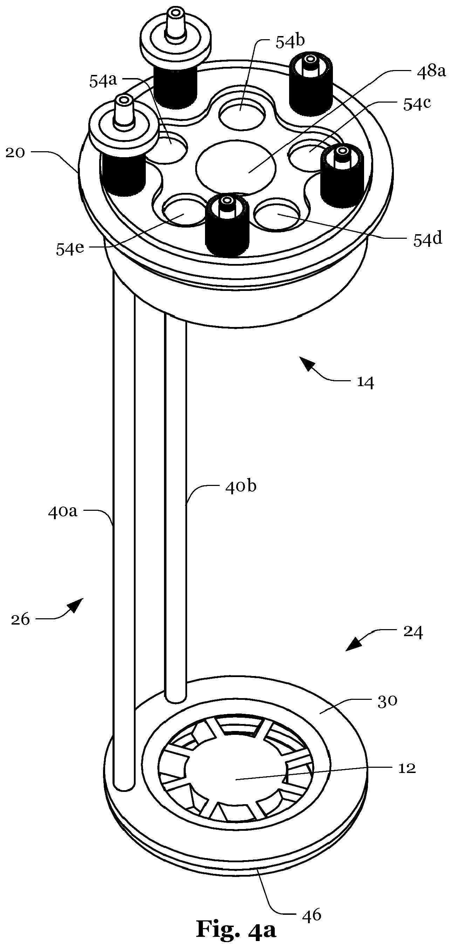

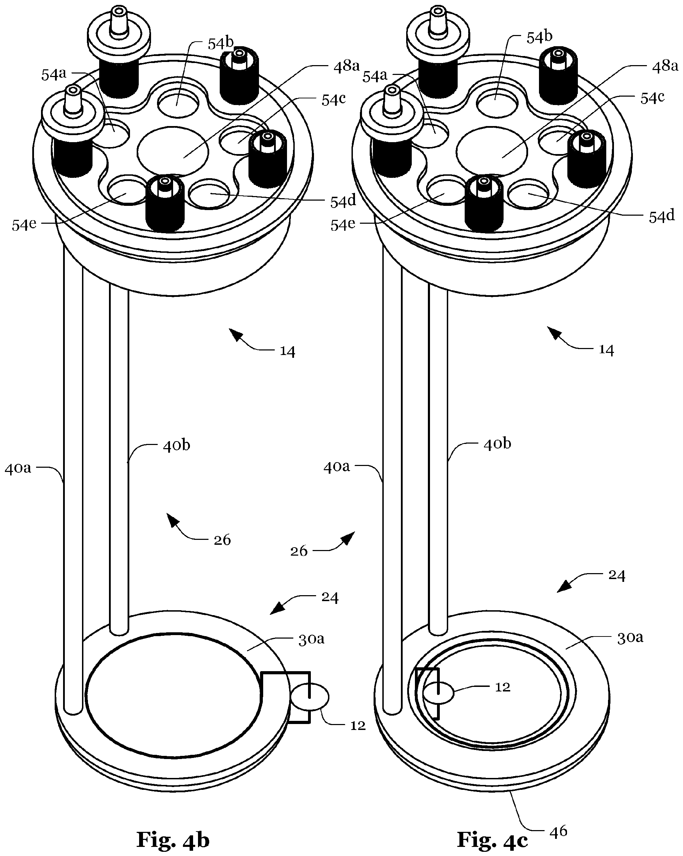

Fig. 4a is a perspective view of the holder 10 of Fig. 2b where the disc-shaped member 20 has a central aperture 48a for a sensor 50 and a multitude of openings 54a, 54b, 54c, 54d and 54e placed around said central aperture 48a. Fig. 4b illustrates a modification of the holder of Fig. 4a, where the bottom section 24 forms an annular guide 30a. The annular guide 30a makes the magnetic agitator 12 rotate along the outside rim of a ring-shaped structure. Fig. 4c illustrates a modification of the holder of Fig. 4b, where the annular guide 30a makes the magnetic agitator 12 rotate along the inner edge of the ring-shaped structure.

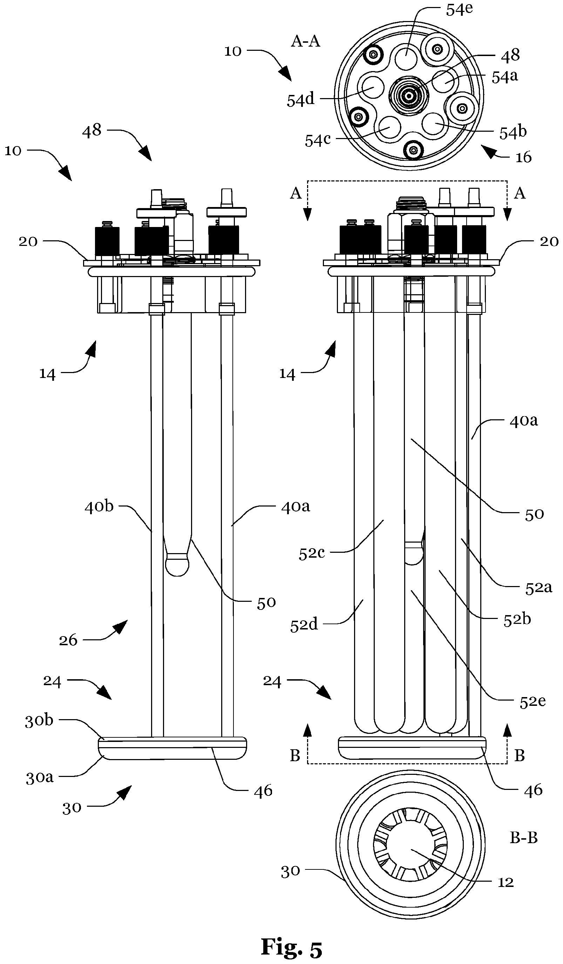

As shown in Fig. 5, the disc-shaped member 20 may comprise an adapter (not shown) to connect one or more gas pipes to the channel 42a and/or the channel 42b. Notably, the gas channels 42a and 42b may be used to feed different gases into the agitated liquid 28. Furthermore, the top section 14 comprises a sensor socket 48 which is formed at a center of the disc-shaped member 20. A sensor 50 which allows measuring the acidity or alkalinity of the liquid 28 is attached to the sensor socket 48 and extends vertically downward from the top section 14. For example, the sensor socket 48 may comprise an aperture 48a in the disc-shaped member 20, wherein the aperture 48a may have an inner thread and the sensor 50 may be threaded into the aperture 48a. The measurements of the sensor 50 may be used to control a rate of CO₂ fed into the liquid 28.

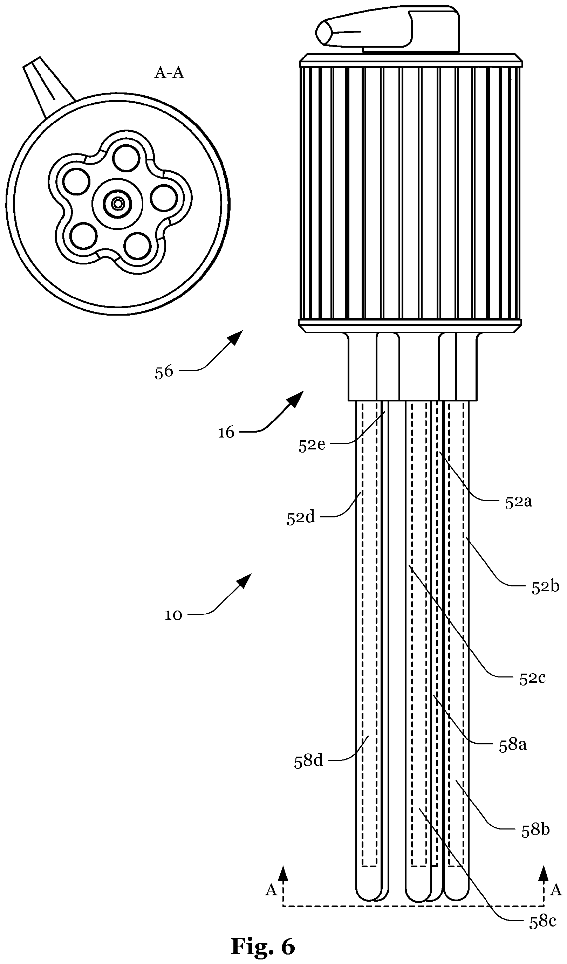

The holder 10 further comprises a plurality of at least partially transparent hollow elongate members 52a, 52b, 52c, 52d and 52e which are attached to the top section 14 and extend through the top section 14 towards the bottom section 24. More particularly, the disc-shaped member 20 has a plurality of openings 54a, 54b, 54c, 54d and 54e and the at least partially transparent hollow elongate members 52a, 52b, 52c, 52d and 52e are inserted into and extend through the openings 54a, 54b, 54c, 54d and 54e. A sealing ring (not shown) may be provided between the transparent hollow elongate members 52a, 52b, 52c, 52d and 52e and an edge of the openings 54a, 54b, 54c, 54d and 54e, respectively. As shown in Fig. 6, the holder 10 may be provided with a cap 56. The cap 56 may be configured to be attachable to and detachable from the top section 14. For example, the cap 56 may comprise an inner thread that engages with an outer thread of the vessel 18 when the cap 56 is attached to the top section 14. The cap 56 may comprise means (not shown) for attaching printed circuit boards (PCBs) 58a, 58b, 58c und 58d to the cap 56. Furthermore, the cap 56 may comprise a power supply, (one or two) adapters for gas pipes and valves for controlling a gas flow through the channel 42a and/or 42b.

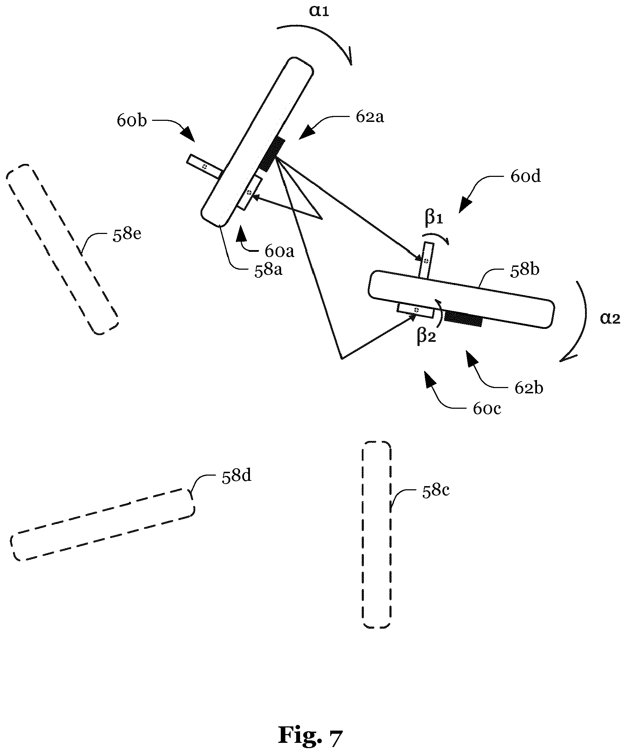

As schematically illustrated in Fig. 7, each printed circuit board 58a, 58b, 58c, 58d and 58e may comprise two detectors 60a and 60b (60c and 60d) mounted to a first side and a second side of the printed circuit board 58a, 58b, 58c, 58d and 58e, respectively, and a source 62a (62b) of electromagnetic radiation mounted to the second side of the printed circuit board 58a, 58b, 58c, 58d and 58e. The printed circuit boards 58a, 58b, 58c, 58d and 58e may be centered at corners of an equilateral polygon (a pentagon) lying in a horizontal plane.

The detectors' 60a and 60b (60c and 60d) orientation and the source's 62a (62b) of electromagnetic radiation orientation may be configured at least partially orthogonal, for example, to be able to distinguish between an intensity of light transmitted directly from the source 62a of electromagnetic radiation to the detector 60d and an intensity of scattered light, indecent on the detector 60c. The orientations (α) of the detectors on the printed circuit boards 58a, 58b, 58c, 58d and 58e may be selected by taking into account geometrical constrains within the hollow elongate members 52a, 52b, 52c, 52d and 52e and may be at least partially orthogonal. Relative angles (β) of detectors 60a, 60b (60c, 60d) and the source 62b (62a) of electromagnetic radiation can be adjusted during measurements by up to 360° rotational degree of freedom. Where a 30° incident angle relative to the radius is preferred in a pentagon arrangement, one of the detectors 60c, 60d is shielded from the direct electromagnetic radiation of the adjacent source 62a of electromagnetic radiation.

Notably, the cap 56 may further comprise all circuitry required to operate the detectors 60a, 60b, 60c and 60d and the sources 62a and 62b of electromagnetic radiation. Furthermore, the cap 56 may comprise a processor for controlling an operation of the device and a (radio) transmitter/receiver which allows collecting data from the device and controlling the device via a (wireless) interface.

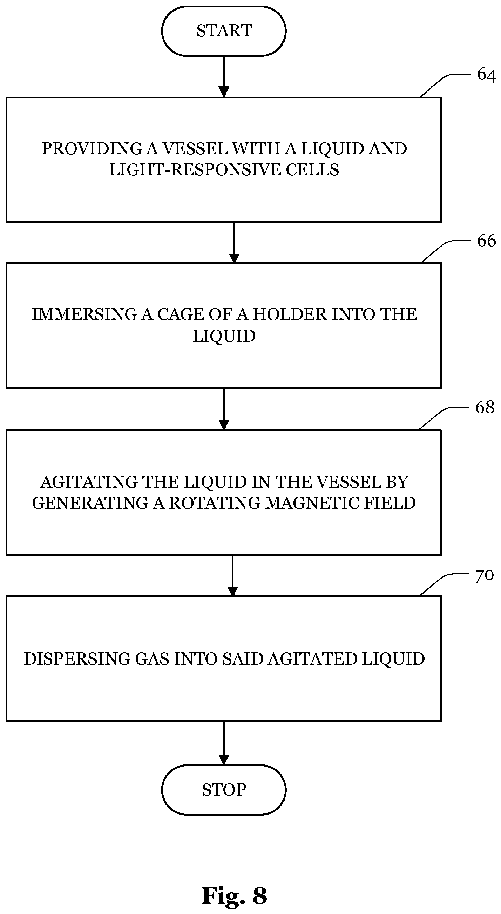

Fig. 8 is a flow chart of a process for cultivating light-responsive cells such as, for example, algae. The process starts at step 64 with providing the vessel 18 with the liquid 28 and light-responsive cells (not shown). The process is continued at step 66 with immersing the cage 30 of the holder 10 into the liquid 28. At step 69, the process is further continued with agitating the liquid 28 in the vessel 18 by generating a rotating magnetic field. During cultivation, step 70 of dispersing a gas into the agitated liquid 28 may be performed.

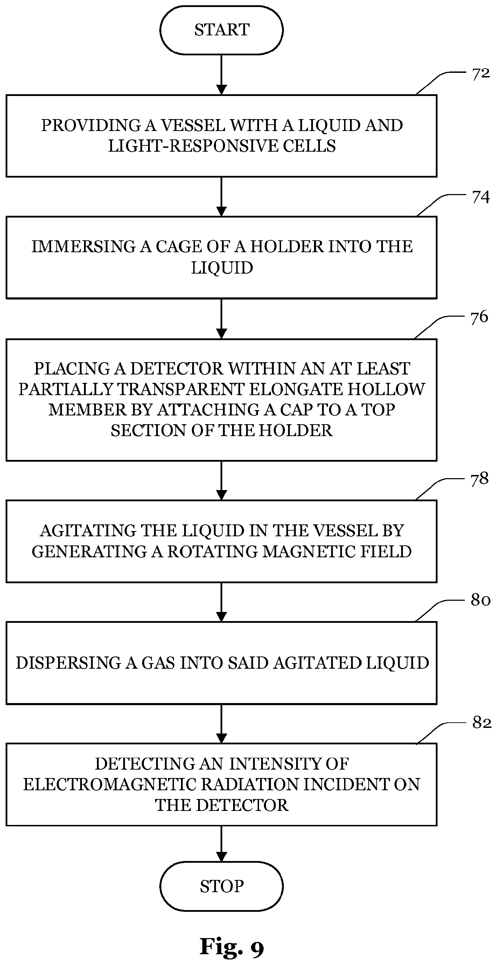

Fig. 9 is a flow chart of another process for cultivating light-responsive cell such as, for example, algae. The process starts at step 72 with providing the vessel 18 with the liquid 28 and light-responsive cells (not shown). The process is continued at step 74 with immersing the cage 30 of the holder 10 into the liquid 28. At step 76, the detectors 60a, 60b, 60c and 60d are placed within the at least partially transparent elongate hollow members 52a, 52b, 52c and 52d and 52e, respectively, by attaching the cap 56 to the top section 14.

When attaching the cap 56 to the top section 14, adapters extending from the top section 14 upwards may be automatically coupled to (corresponding) adapters extending from the cap 56 downwards . I.e., instead of establishing the connections one after the other, the connections are established in parallel, thereby saving time and labor. At step 78, the process is further continued with agitating the liquid 28 in the vessel 18 by generating a rotating magnetic field.

During cultivation, step 80 of dispersing a gas into the agitated liquid 28 and step 82 of detecting an intensity of electromagnetic radiation incident on the detectors 60a, 60b, 60c and 60d may be performed.

### Reference signs list

- 10: holder
- 12: magnetic agitator
- 14: top section
- 16: aperture
- 18: vessel
- 18a: neck
- 20: disc-shaped member
- 20a: fastener
- 22: rim
- 24: bottom section
- 26: elongate middle section
- 28: liquid
- 30: cage
- 30a: annular guide
- 30a: ring-shaped element
- 30b: ring-shaped element
- 30c: ring-shaped section
- 30d: ring-shaped section
- 30e: ring-shaped section
- 32: chamber
- 34: opening
- 36: opening
- 38a: stirrer device
- 38b: stirrer device
- 40a: rod
- 40b: rod
- 42a: channel
- 42b: channel
- 44: ring-shaped channel section
- 46: ring-shaped element
- 48: socket
- 50: sensor
- 52a: hollow elongate member
- 52b: hollow elongate member
- 52c: hollow elongate member
- 52d: hollow elongate member
- 52e: hollow elongate member
- 54a: opening
- 54b: opening
- 54c: opening
- 54d: opening
- 54e: opening
- 56: cap
- 58a: printed circuit board
- 58b: printed circuit board
- 58c: printed circuit board
- 58d: printed circuit board
- 60a: detector
- 60b: detector
- 60c: detector
- 60d: detector
- 62a: source
- 62b: source
- 64: process step
- 66: process step
- 68: process step
- 70: process step
- 72: process step
- 74: process step
- 76: process step
- 78: process step
- 80: process step
- 82: process step

## Claims

1. A holder (10) for a magnetic agitator (12), said holder (10) comprising:
a top section (14) which is configured to be attached to a vessel (18), wherein the top section (14) is preferably configured to be attached to an aperture (16) of said vessel;
a bottom section (24) which is configured to be immersed into a liquid (28) contained in said vessel (18); and
an elongate middle section (26) arranged between and connecting said bottom section (24) and said top section (14);
wherein said bottom section (24) forms at least one of a cage (30) and an annular guide (30a) for said magnetic agitator (12).

2. The holder (10) of claim 1, further comprising:
a channel (42a, 42b) for feeding a gas into said agitated liquid (28);
wherein, preferably, said gas is fed into said agitated liquid (28) through at least one of a porous material and a ring-shaped element (44) made of a porous material.

3. The holder (10) of claim 1 or 2, further comprising:
means for generating a rotating electromagnetic field for causing a rotation of said magnetic agitator (12);
wherein, preferably, an axis through a center of rotation of said rotating electromagnetic field extends through a center of said cage (30) and/or said annular guide (30a).

4. The holder (10) of claim 2 or 3,
wherein said elongate middle section (26) comprises at least one of a hollow bar and a hollow rod (40a, 40b); and
wherein, preferably, at least one of said channel (42a, 42b) and a line for supplying energy to said means for generating a rotating electromagnetic field is arranged, at least section-wise, within said hollow bar or said hollow rod (40a, 40b).

5. The holder (10) of any one of claims 1 to 4, wherein said top section (24) comprises a sensor socket (48) for holding a sensor (50) in a position in which said sensor (50) is immersed in said liquid (28),
wherein, preferably, said top section (24) comprises a disc-shaped member (20) and said sensor socket (48) is formed at a center of said disc-shaped member (20).

6. The holder (10) of any one of claims 1 to 5, further comprising:
an at least partially transparent hollow elongate member (52a, 52b, 52c, 52d, 52e);
wherein said at least partially transparent hollow elongate member (52a, 52b, 52c, 52d, 52e) is configured to be attachable to and detachable from said top section (14);
wherein said at least partially transparent hollow elongate member (52a, 52b, 52c, 52d, 52e) extends from said top (14) section towards said bottom section (24) when said at least partially transparent hollow elongate member (52a, 52b, 52c, 52d, 52e) is attached to said top section (14); and
wherein, preferably, said at least partially transparent hollow elongate member (52a, 52b, 52c, 52d, 52e) extends through said top section (14).

7. The holder (10) of claim 6, further comprising:
a cap (56) which is configured to be attachable to and detachable from said top section (14);
wherein said cap (56) comprises a detector (60a, 60b, 60c, 60d) which is arranged within said at least partially transparent hollow elongate member (52a, 52b, 52c, 52d, 52e) when said cap (56) is attached to said top section (14).

8. The holder (10) of claim 7, further comprising:
another at least partially transparent hollow elongate member (52a, 52b, 52c, 52d, 52e);
wherein said other at least partially transparent hollow elongate member (52a, 52b, 52c, 52d, 52e) is configured to be attachable to and detachable from said top section (14);
wherein said other at least partially transparent hollow elongate member (52a, 52b, 52c, 52d, 52e) extends from said top section (14) towards said bottom section (24) when said other at least partially transparent hollow elongate member (52a, 52b, 52c, 52d, 52e) is attached to said top section (14);
wherein, preferably, said other at least partially transparent hollow elongate member (52a, 52b, 52c, 52d, 52e) extends through said top section (14); and
wherein said cap (56) comprises a source (62a, 62b) of electromagnetic radiation.

9. The holder (10) of claim 8, wherein said source (62a, 62b) of electromagnetic radiation is arranged within said other at least partially transparent hollow elongate member (52a, 52b, 52c, 52d, 52e) when said cap (56) is attached to said top section (14).

10. The holder (10) of claim 9, wherein
said detector (60a, 60b) is mounted to a first side of a printed circuit board (58a, 58b, 58c, 58d) which comprises another source (62a, 62b) of electromagnetic radiation mounted to a second side of said printed circuit board (58a, 58b, 58c, 58d); and
said source (62a, 62b) of electromagnetic radiation is mounted to a second side of another printed circuit board (58a, 58b, 58c, 58d) which comprises another detector (60a, 60b, 60c, 60d) mounted to a first side of said other printed circuit board (58a, 58b, 58c, 58d).

11. The holder (10) of claim 10, wherein said printed circuit boards (58a, 58b, 58c, 58d) each comprise two detectors (60a, 60b, 60c, 60d).

12. The holder (10) of claim 10 or 11,
wherein said holder (10) comprises at least three substantially identical at least partially transparent hollow elongate members (52a, 52b, 52c, 52d, 52e);
wherein said three substantially identical at least partially transparent hollow elongate members (52a, 52b, 52c, 52d, 52e) are arranged at corners of an equilateral polygon lying in a horizontal plane;
wherein, preferably, each of said at least partially transparent hollow elongate members (52a, 52b, 52c, 52d, 52e) houses a detector (60a, 60b, 60c, 60d) and a source (62a, 62b) of electromagnetic radiation, preferably one detector (60a, 60b, 60c, 60d) and two sources (62a, 62b) of electromagnetic radiation.

13. A device for agitating a liquid (28) contained in a vessel (18) and dispersing a gas into said agitated liquid (28), comprising:
said vessel (18); and
a holder (10) of any one of claims 1 to 12;
wherein said holder (10) is attached to an aperture (16) of said vessel (18).

14. A method of cultivating light-responsive cells, comprising:
providing (64) a vessel (18) with a liquid (28) and said light-responsive cells;
immersing (66) at least one of said cage (30) and said annular guide (30a) of a holder (10) according to any one of claims 1 to 12 into said liquid (28);
agitating (68) said liquid (28) in said vessel (18) by generating a rotating magnetic field; and
dispersing (70) a gas into said agitated liquid (28);
wherein, preferably, said gas comprises CO₂.

15. A method of cultivating light-responsive cells, comprising:
providing (72) a vessel (18) with a liquid (28) and said light-responsive cells;
immersing (74) at least one of said cage (30) and said annular guide (30a) of a holder (10) according to any one of claims 7 to 12 into said liquid;
placing (76) said detector (10) within said at least partially transparent elongate hollow member (52a, 52b, 52c, 52d, 52e) by attaching said cap (56) to said top section (14);
agitating (78) said liquid (28) in said vessel (18) by generating a rotating magnetic field;
dispersing (80) a gas into said agitated liquid (28);
wherein, preferably, said gas comprises CO₂; and
detecting (82) an intensity of electromagnetic radiation incident on the detector (60a, 60b).
